# EUROPEAN PATENT APPLICATION

(11) **EP 4 850 720 A1**
(43) Date of publication of application: **09.12.2026**
(21) Application number: 25180399.5
(22) Date of filing: 03.06.2025
(51) Int. Cl.: A61M 5/20, A61M 5/24, A61M 5/31, A61M 5/315, A61M 5/32

(54) **DRIVE UNIT OF A DRUG DELIVERY DEVICE FOR DISPENSING PREDEFINED VARIABLE DOSES**

(71) Applicant: Ypsomed AG, 3401 Burgdorf (CH)
(72) Inventor: Bosshard, Simon Martin, 3324 Hindelbank (CH); Eraslan-Atasoy, Fadime, 4142 Münchenstein (CH); Müller, Ivana, 3427 Utzenstorf (CH); Collings, Ralph Donald Quentin, Chepstow, NP16 7NN (GB); Fogg, Oliver David, Bristol, BS2 0EH (GB); De Saulles, Jerome Antony, Bristol, BS3 1HH (GB); McLennan, Cal, Chepstow, NP16 7FQ (GB); Perkins, Hugh Thomas, Bristol, BS3 2BN (GB)

(57) **Abstract**

The present invention relates to a drive unit of a drug delivery device for dispensing predefined doses of a liquid drug, releasably attachable to a reservoir. The drive unit comprises a drive unit housing defining a longitudinal direction, a plunger rod movable inside the drive unit housing in a dispensing direction to dispense the liquid drug and a mechanical drive, preferably a torsion spring, arranged inside the drive unit housing for moving, when loaded, the plunger rod in the dispensing direction; and a dose setting mechanism comprising a dose setting element and a trigger element, wherein a loading stroke of the trigger element causes the dose setting mechanism to load the mechanical drive. According to the invention, the dose setting element is movable to change a stroke distance of the plunger rod such that the predefined dose is changeable while the loading stroke of the trigger element remains the same.

## Description

### FIELD OF THE INVENTION

The present invention relates to a drive unit of a drug delivery devices or medicament delivery devices for injecting, delivering, administering, infusing or dispensing substances and/or liquids such as insulin, hormone preparations or vaccines. It departs from a drug delivery device comprising a preferably reusable drive unit and a preferably disposable reservoir unit configured for releasable attachment to the drive unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a stopper in a container barrel, wherein the drive spring may have to be charged or strained manually prior to injection of a dose. A manually powered delivery drive requires a user to manually provide the energy to move the stopper, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the stopper manually or by pressing a release button of an automatic injection device. Automatic injection devices may comprise an electronic dose dial mechanism to automatically set a dose.

In the market, reusable pens and disposable pens are available. Normally, for both, the user can inject several doses of a medicament until the cartridge is empty. For disposable pens, the user then discards the entire pen device. For reusable pens, the user only discards the empty cartridge and replaces it with a new cartridge. This allows to reduce waste and to handle and dispose of the injection pen parts differently.

Also known is the use of autoinjectors with syringes. Autoinjectors usually comprise a body for housing a syringe as well as a drive mechanism to move the stopper of the syringe upon actuation of the autoinjector. The drive mechanism typically comprises a source of drive, such as an electric motor or a mechanical spring for moving a transfer element, for example a plunger rod, which acts on the stopper of the syringe. The prefilled syringe has a needle or cannula that is permanently attached to a first end of a syringe barrel or reservoir that is sealed at the opposing end by the stopper.

For safety and hygiene reasons it is desirable that the needle does not protrude from the housing of the autoinjector except for the time when the needle is used for injection of a medicament. Thus, either the autoinjector moves the needle out of the housing for the injection and back into the housing after injection or the autoinjector includes a needle cover which may be moved to unsheathe the needle for injection, and which may be moved back to a needle covering position after the injection.

The majority of autoinjectors are configured as single use devices which incorporate both the syringe and the drive mechanism in the same housing. Such devices are usually disposable for hygiene reasons. Disposable autoinjectors comprising an electric actuator or an electronic control require a source of energy which is usually in the form of a battery. However, in this case, the autoinjectors should not be disposed of in the regular waste but must be subjected to special disposal or to recycling, which is an additional burden to the patient. Further, disposing of a battery, motor and/or electronics after a single use is a waste of resources and increases the costs of the therapy. Disposable autoinjectors comprising a mechanical drive, like a compression spring, are easier to dispose of, however it is also a waste of resources to dispose of the compression spring after a single use, which also increases the costs of the therapy.

To account for the need to handle and dispose of the autoinjector parts differently, semi-reusable autoinjectors have been developed. Such autoinjectors typically comprise a reusable drive unit and a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection.

A big problem for semi-reusable mechanical autoinjectors is that the compression spring must be recompressed or re-loaded after each injection, which can require a high force and accuracy, which can be challenging for the user, especially for older patients. Another problem is that with known autoinjector devices, it is not possible to change the dose, because with every injection stroke the whole content of the syringe is injected. Therefore, it is also necessary to change the syringe and load the compression spring after each injection, which can be annoying and time consuming for the user.

EP 2 618 870 A2 discloses a semi-reusable autoinjector comprising a reusable drive unit with a mechanical drive mechanism and a disposable syringe unit. When the syringe unit is inserted in the drive unit, the syringe unit rotates a winder element that is operationally connected to an input transmission train for charging the mechanical drive mechanism. CN 217489449 U discloses a semi-reusable autoinjector comprising a drive unit and a detachable syringe unit. During the insertion the stopper of the syringe contacts the plunger and moves the plunger to a retracted position to load the mechanical drive. WO 2018/091916 A1 discloses further semi-reusable autoinjector with a reusable drive unit and a disposable reservoir unit that can be releasably connected to the drive unit. Before the reservoir unit is connected to the drive unit, a compression spring for driving a plunger to expel the medicament must be loaded by inserting the drive unit in a special reloading station that compresses the spring. US 2015/15117592 A discloses another semi-reusable autoinjector differing from WO 2018/091916 A1 in that the compression spring is charged by pressing the driving assembly against a flat surface, like a desk or a wall. WO 2014/029621 A1 discloses another semi-reusable autoinjector comprising a front housing and a rear housing, releasably connectable to each other, and a replaceable container assembly. The rear housing comprises a reloadable drive mechanism for acting on a stopper of the container assembly for expelling a medicament. A needle shield remover that is used to remove a needle shield of the container assembly can also be used to reload the drive mechanism.

These known semi-reusable autoinjectors are sometimes difficult to assemble and there is a risk that the syringe is inserted in a twisted or turned orientation so that the plunger exerts the drive force on the syringe piston in a non-perpendicular direction, which can destroy the syringe or lead to an amount of injected medicament that deviates from the desired amount. In addition, these known devices cannot overcome the problems that the dose is fixed and that the syringe must be changed after each injection.

### DESCRIPTION OF THE INVENTION

It is an objective of the present invention to provide a drive unit of a drug delivery device with a mechanical drive that allows a user to load the drive unit by tensioning the mechanical drive in an easy and reliable way and to change the dose to be injected.

This objective is achieved by the features of the independent claims. Preferred embodiments are evident from the dependent claims.

The invention relates to a drive unit of a drug delivery device for dispensing predefined doses of a liquid drug. A reservoir is releasably attachable to the drive unit to provide a complete drug delivery device. It should be clear that the reservoir can be insertable into the drive unit or that it can be attachable to a distal end of the drive unit. In both cases the reservoir is releasably attachable to the drive unit. The reservoir can be insertable into the drive unit and/or attachable to the drive unit from a distal end of the drive unit either by a linear movement in a longitudinal direction or by a rotational movement. The reservoir can also be arranged in a reservoir holder that is insertable into the drive unit and/or attachable to the drive unit. The reservoir holder can be disposable for safety reasons or reusable for environmental reasons.

The reservoir is preferably a cartridge and comprises a connecting portion at the distal end adapted to be connected to an injection needle. Preferably, the reservoir is a 1 ml or 3 ml cartridge. The needle can be a normal pen needle or a safety needle. For hygiene reasons, the needle needs to be discarded after each injection, so that for a new injection, a new needle must be attached to the reservoir. Preferably, the needle can be connected to the connecting portion of the reservoir by a snap-fit connection. Preferably, the needle can be placed in a needle container prior to the connection with the reservoir and the snap-fit connection can be established by pushing a distal end of the reservoir into the needle container. Preferably, the liquid drug is a medicament for obesity treatment.

Preferably, the attachment between the reservoir and the drive unit is non-movable, which means that during an injection the reservoir and hence the needle does not move relative to the drive unit housing, so that the user must insert or penetrate the injection needle into the skin at a desired injection site by a manual insertion force. Preferably, the drive unit is reusable so that it can be reused with a new reservoir after the currently attached reservoir is empty. However, it is also possible to provide the drive unit as a disposable part that is disposed of after the reservoir is empty. Preferably, the reservoir is used for several injections before it is empty, however it is also possible to inject the whole content of the reservoir in one stroke, if needed.

The drive unit comprises a drive unit housing defining a longitudinal direction, a plunger rod movable inside the drive unit housing in a dispensing direction to dispense the liquid drug and a mechanical drive, preferably a torsion spring or a compression spring, arranged inside the drive unit housing for moving, when loaded, the plunger rod in the dispensing direction. The plunger rod can also be referred to as piston rod or drive element. The mechanical drive can directly contact the plunger rod to move the plunger rod in the dispensing direction or the plunger rod can be coupled to the plunger rod over one or more additional components, for example a drive nut. The longitudinal direction can define a longitudinal axis of the drive unit, if the drive unit is substantially cylindrical. The longitudinal direction of the drive unit is also considered as the longitudinal direction of the entire drug delivery device. The dispensing direction can preferably be the longitudinal direction and more preferably a distal direction. The drive unit housing can comprise only one part with a sleeve structure. The drive unit housing can also comprise two or more parts that together form a sleeve structure. A separation between the parts can run in the circumferential direction of the sleeve, in other words the parts can be sleeves with a shorter length, or transversely thereto, in other words the parts can be half shells or partial shells which together form a sleeve structure. In this alternative, the two or more drive unit housing parts can be connected mechanically, for example with screws or latches, or they can be bonded to each other, for example by gluing or welding. For loading, the mechanical drive is preferably compressed in a proximal direction, hence a distal end of the mechanical drive is moved in the proximal direction while the proximal end of the mechanical drive is fixed. Preferably the plunger rod is latched in the proximal position of the plunger rod, in which the mechanical drive is loaded, particularly in which the drive spring is compressed, so that the mechanical drive stays in the loaded position, making the drive unit ready to perform an injection.

The drive unit further comprises a dose setting mechanism. The dose setting mechanism comprises a dose setting element and a trigger element, wherein a loading stroke of the trigger element causes the dose setting mechanism to load the mechanical drive. The dose setting element allows setting a predefined dose that will be injected. With other words, when the trigger element is moved to perform a loading stroke, the dose setting mechanism loads the mechanical drive to inject a dose set by the dose setting element. Preferably, the dose setting mechanism sets the same dose for the entire reservoir attached to the drive unit. This means that the dose is not changed until the reservoir is empty and a new reservoir is attached to the drive unit. Hence, the user does not have to set the dose before each injection, so that the injection includes one user step less, which makes it easier and more comfortable to use the injection device for frequent injections with the same dose of the same medicament. For the new reservoir, the dose can be changed by the dose setting mechanism or it can be kept in the same configuration as for the previous reservoir, so that a different or the same dose will be injected for the new reservoir. Hence, the dose setting mechanism sets a variable fixed dose.

According to the invention, the dose setting element is movable to change a stroke distance of the plunger rod such that the predefined dose is changeable while the loading stroke of the trigger element remains the same. With other words, a movement of the dose setting element makes it possible to inject different doses for the same loading stroke of the trigger element. A drug delivery device offers a range of possibilities to set or alter the dose that will be injected without changing the loading stroke of the trigger element. In general, it is on the one hand possible to change the amount the mechanical drive is loaded with the loading stroke, with other words to change the interaction between the loading stroke and the loading of the mechanical drive. On the other hand, it is alternatively possible to fully load the mechanical drive with the loading stroke and to alter the distance the plunger rod advances due to the bias of the mechanical drive, with other words to change the interaction between the mechanical drive and the stroke distance of the plunger rod. Preferably, the dose setting element remains in one position so that the same dose is injected, until the dose setting element is manually moved to another position by the user. With other words, preferably the set dose remains the same until the user actively moves the dose setting element.

An advantage of the drive unit according to the invention is that the user can execute the same loading stroke of the trigger element and hence the same user step regardless of the set dose, which makes the drive unit and hence the drug delivery device particularly easy and comfortable to use. Hence, the drug delivery device is particularly user-friendly and provides patients, particularly obese patients, with confidence to deliver their doses successfully. Another advantage is that the dose is fixed for the entire reservoir life, so that the user must set the dose a lot less frequently, in fact only when the user wants to change the dose.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "distal" is meant to refer to the direction or the end of the drive unit or drug delivery device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion device" refers to a device with a cannula or needle that remains in the skin of the patient for a prolonged period of time, for example, several hours.

In the claims, the word "comprising" or "comprises" does not exclude other elements or steps, and the indefinite article "a" or "an" does not exclude a plurality. For example, "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

In a preferred embodiment, the mechanical drive is fully loaded with each loading stroke of the trigger element and the dose setting element changes the stroke distance of the plunger rod by altering the transmission between the mechanical drive and the plunger rod. This means that the mechanical drive is fully loaded with each loading stroke and fully unloaded during each injection, but not the entire bias force of the mechanical drive is translated or transferred into a dispensing movement of the plunger rod. With other words, a different amount of the full drive stroke of the mechanical drive is transferred depending on the set dose. This has the advantage that the loading stroke of the trigger element requires the same force regardless of the set dose. The mechanical drive being unloaded after each injection further has the advantage that the danger of a tensioned spring during storage is prevented. In this preferred embodiment, the user loads the mechanical drive by moving the trigger element for each injection.

In a preferred embodiment, the drive unit further comprises a drive nut and a stepped drum for transferring force from the mechanical drive to a movement of the plunger rod in the dispensing direction. In this preferred embodiment, the stepped drum is the dose setting element and comprises teeth of different longitudinal length. The stepped drum is movable relative to the drive nut in the longitudinal direction in such a way that the number of teeth of the stepped drum engaging with teeth of the drive nut is altered. This way a different amount of the full dispensing stroke is transferred to the drive nut and hence to the plunger depending on the longitudinal position of the stepped drum. With other words a full rotation of the stepped drum causes a partly rotation of the drive nut. The drive nut preferably comprises an inner thread to advance the plunger rod in the dispensing direction upon a rotation of the drive nut. The mechanical drive is preferably a torsion spring. The stepped drum can also be referred to as Leibnitz wheel.

Preferably, the stepped drum comprises a gap between at least two adjacent teeth such that the drive nut can freely rotate, when the gap faces the drive nut. The gap is then positioned relative to the drive nut in such a way, that the teeth of the drive nut can pass through the gap, which enables the drive nut to rotate freely. This can be used during reset of the drive unit respectively the drug delivery device, when the plunger rod is pushed back in the proximal direction. The gap has the advantage, that the user does not need a high force to push the plunger rod in the proximal direction, because due to the gap, the plunger rod is disconnected from the stepped drum and therefore from the mechanical drive, with other words the user does not have to provide the force to load the mechanical drive when pushing back the plunger.

In a preferred embodiment, the trigger element is a cap of the drive unit and the dose setting mechanism is adapted to load the mechanical drive upon a distal movement of the cap. The distal movement of the cap occurs when the user removes the cap from the drive unit. The removal of the cap can be the first user step the user performs every time he wants to make an injection. Hence, the cap respectively the cap removal is very suitable as trigger element for the dose setting mechanism. With other words is the loading of the mechanical drive a by-product of the cap removal. This has the advantage that no additional component or user step is necessary to load the mechanical drive. A maximum force required for loading the mechanical drive by removing the cap can be less than 25 N to make the drive unit comfortable to use. After the injection is finished and the user has pulled off the needle from the reservoir, the user can push the cap back onto the drive unit. Hence, the cap is reusable for a plurality of injections.

Preferably, the mechanical drive is arranged on a priming rod coupled to a priming gear, the priming rod being rotatable to load the mechanical drive. The cap is coupled to a rack cooperating with a helical gear, the helical gear cooperating with the priming gear. Preferably, the rack comprises teeth and the helical gear comprises helical teeth to cooperate with the teeth of the rack to translate or transfer the axial movement of the cap to a rotation of the helical gear. Preferably, the helical gear further comprises regular teeth to cooperate with the priming gear to translate the rotation of the helical gear to a rotation of the priming gear. Hence, in this embodiment, the axial movement of the cap is translated into a rotation of the priming rod.

Preferably, the priming gear and/or the priming rod has a ratchet feature so that the priming gear and the priming rod rotate together when the priming gear is rotated in a first direction and the priming gear rotates without rotating the priming rod when the priming gear rotates in an opposite direction. This way it is possible to load the mechanical drive when the cap is removed but to decouple the movement of the cap from the mechanical drive, when the cap is reattached after the injection. Additionally or alternatively, the priming rod is rotationally coupled to a check tab movable together with the stepped drum and the plunger rod comprises a protrusion on its proximal end that is adapted to collide with the check tab when a dose remaining in the reservoir is insufficient for the set dose. The proximal plunger protrusion moves in the distal direction, when a dose is injected and reaches the check tab, when the reservoir is almost empty after several injections. The proximal plunger rod protrusion can additionally provide a visual indicator that the remaining dose is insufficient, in that the proximal plunger rod protrusion might only be visible to the user, when the plunger rod reaches its distal position.

In a preferred embodiment, the drive unit comprises a trigger button to start the injection, preferably on a proximal end of the drive unit. The user can press the trigger button to start the injection. The trigger button gives the user an enhanced perception of control as the user can decide when exactly he wants to start the injection. In some embodiments, it can be necessary to hold the trigger button down during the entire injection process, particularly until an end of dose feedback is received, and in other embodiments the trigger button is only pressed once to start the injection, and the user can then release the trigger button without stopping the injection. Preferably the trigger button comprises a ramped proximal surface. The ramped proximal surface makes it more comfortable to press the trigger button. In addition, it can help the user to hold the drug delivery device in a correct orientation, in which it can for example be easier to reach and move the dose setting element or to see a visual indicator of the drug delivery device. Additionally or alternatively, the trigger button comprises a rotational end stop abutting the stepped drum after a full rotation of the stepped drum. This ensures that the stepped drum does not rotate further than a full rotation, which would unintentionally dispense more medicament than the set dose.

Preferably, the priming rod comprises a protrusion that limits the trigger button from being pressed before the mechanical drive is loaded. With other words, the projection interlocks the trigger button between doses. The trigger button can be pressed only after the priming rod is rotated and the protrusion no longer abuts the trigger button. This prevents the user from releasing the plunger rod during loading of the mechanical drive, which could be dangerous for the user and/or damage the drive unit. Instead of the protrusion, the priming rod could also comprise a flange with a recess, wherein the flange blocks the trigger button before the mechanical drive is loaded and the recess enables the trigger button to pass the flange only after the priming rod is rotated. Additionally or alternatively the priming rod comprises a protrusion that locks the trigger button in a pressed-in position during the injection, with other words during dose dispensing. Hence, the user presses the trigger button only once to start the injection and can then release the trigger button. This makes the drive unit more comfortable to use and prevents the injection of partial doses.

In a preferred embodiment, the mechanical drive is partly or fully loaded with each loading stroke of the trigger element dependent on the position of the dose setting element. Hence, the movement of the dose setting element changes how much the mechanical drive is loaded with the loading stroke of the trigger element, while the loading stroke of the trigger element remains the same regardless of the set dose. After the injection, the mechanical drive is fully unloaded. This makes the drug delivery device easy and comfortable to use for the user, because the user can fulfill the same user step regardless of the set dose. The mechanical drive being unloaded after each injection further has the advantage that the danger of a tensioned spring during storage between the injections is prevented.

In another preferred embodiment, the trigger element is a needle cover of the drive unit movable between an extended position in which it covers the needle and a retracted position in which it reveals the needle. The movement of the needle cover allows switching between a safety state in which the needle is covered and does not protrude from the reservoir unit and an injection state in which the needle is uncovered and protrudes from the reservoir unit. Preferably, the needle cover is movable along the longitudinal axis respectively in the longitudinal direction and/or guided by the drive unit housing. The dose setting mechanism is adapted to load the mechanical drive by a proximal movement of the needle cover from the extended position to the retracted position. The movement of the needle cover is necessary for every injection and is therefore suitable as trigger element to load the mechanical drive. This has the advantage that no extra component or user step is necessary to load the mechanical drive. Preferably, the needle cover moves from the extended position to the retracted position by pressing the medicament delivery device and hence the needle cover against the skin at a desired injection site which pushes the needle cover into the drive unit. Preferably, the needle cover is a needle cover sleeve. In this preferred embodiment, the needle cover can additionally be used as release element to release the plunger rod, so that no extra elements, such as a release button or injection button, and no extra user steps, such as pressing an additional release button or injection button, are necessary. Preferably, the needle cover can be biased towards the extended position, for example with a needle cover spring. Preferably, after the injection the needle cover can be biased into a final extended position, particularly distal from the initial extended position, in which the needle cover is locked and can hence not move back into the reservoir unit to provide needle protection.

Preferably, the needle cover is movable from the extended position to an intermediate position in which it still covers the needle by attaching the needle to the reservoir and from the intermediate position to the retracted position by pressing the needle cover against the skin. This division of the loading stroke reduces the stroke distance and/or the force that the user must overcome while pressing the needle cover against the skin. The needle attachment is also necessary for every injection and is hence also suitable as trigger element. Preferably, the needle attachment can provide approximately 25% of the loading stroke and the pressing against the skin can provide approximately 75% of the loading stroke. The force required during skin contact can then be below 10 N, which makes the drive unit comfortable to use.

In a preferred embodiment, the dose setting element is a priming sleeve movable together with the needle cover in the proximal direction, wherein the priming sleeve comprises a thread for translating its proximal movement to a rotation of a loading sleeve that loads the mechanical drive. The priming sleeve is movable relative to the needle cover in the longitudinal direction in such a way that a distance between a proximal end of the needle cover in the extended position and the priming sleeve is altered. This means that when the needle cover moves in the proximal direction from the extended position to the retracted position, depending on the set dose there is an altering distance in which the needle cover does not engage and therefore does not move the priming sleeve. Over this initial distance, the mechanical drive is not loaded so that the final loading of the mechanical drive differs depending on this initial distance. However, the loading stroke of the trigger element, hence the needle cover, is the same regardless of the set dose. The loading of the mechanical drive with the priming sleeve and the loading sleeve further has the advantage that the force required to load the mechanical drive is reduced by way of translating the longitudinal movement of the priming sleeve to the rotating movement of the loading sleeve.

Preferably, the drive unit further comprises a plunger rod shuttle comprising a thread to cooperate with the loading sleeve and engaging with the plunger rod to translate or transfer rotation of the loading sleeve to a movement of the plunger rod in the dispensing direction. This enables an easy way to translate the rotating movement of the loading sleeve into an axial movement of the plunger rod in the dispensing direction. In this embodiment, the loading sleeve has an inner thread to cooperate with the plunger shuttle and an outer thread to cooperate with the priming sleeve. Differing pitches between the inner thread and the outer thread can reduce the force the user has to exert to move the needle cover to load the mechanical drive. Preferably, the outer pitch can be greater than the inner pitch. For example, the inner pitch can be 14 mm, and the outer pitch can be 42 mm.

Preferably, the drive unit further comprises a plunger rod reset sleeve movable between a first extended position and second retracted position by inserting the reservoir. In the first position, the plunger rod is separated from the plunger shuttle and in the second position, the plunger rod is coupled to the plunger shuttle. The plunger rod reset sleeve comprises a helical ramp to rotate the plunger rod to engage and disengage the plunger rod while moving between the first position and the second position. The first position is distal to the second position, so that the reservoir pushes the plunger rod reset sleeve in the proximal direction into the second position. In the first position, the drive unit can be reset after the reservoir is empty and has been removed. Due to the separation, the plunger rod can be pushed back inside the drive unit to reset the drive unit for the next reservoir. In the second position, the plunger rod shuttle moves the plunger rod to dispense medicament from the reservoir.

In a preferred embodiment, the mechanical drive is fully loaded with each loading stroke of the trigger element and the dose setting mechanism changes the dispensing distance of the plunger rod by mechanically stopping the plunger rod after different dispensing distances. The mechanical drive therefore stays in a partly loaded state after the injection until the last dose is injected and the reservoir is empty. With other words, the mechanical drive reaches its unloaded or relaxed state only when the reservoir is empty. Hence, the mechanical drive is loaded once for the entire reservoir life, meaning that the user only performs one loading stroke of the trigger element per reservoir. The mechanical drive is preferably loaded by pushing the plunger rod in the proximal direction, for example when the user inserts a new reservoir into the drive unit.

Preferably, the dose setting element is formed by at least two tracks on the plunger rod extending in the longitudinal direction. The tracks each comprise a plurality of end stops arranged with the same distance in longitudinal direction to each other. The drive unit further comprises a stop element connected to the drive unit housing and interacting with the end stops such that the plunger rod is mechanically stopped at each end stop. The end stops have a different distance between them for each track and the plunger rod and/or the stop element is rotatable to align the end stop with different tracks. So, when the plunger rod and the stop element are rotated relative to each other, the plunger rod is stopped after different distances of plunger rod travel, so that a different dose is injected. In this embodiment, the mechanical drive is preferably a compression spring.

Preferably, the stop element has a flat proximal surface providing a stop feature when the plunger rod moves in the dispensing direction and a ramped distal surface enabling the plunger rod to pass the stop element in the proximal direction during reset of the drive unit. This configuration of the stop element makes it easy for the user to push the plunger rod back in the proximal direction when a new reservoir is inserted into the drive unit.

Preferably, the drive unit further comprises a needle cover movable between an extended position in which it covers the needle and a retracted position in which it reveals the needle. The trigger button is movable from a retracted position in which it is fixed and an extended position in which the user can press the trigger button. With other words, the trigger button must be in its extended position for the user being able to press the trigger button to start the injection. In this embodiment, the movement of the needle cover is coupled to the movement of the trigger button such that the trigger button moves from the first position to the second position when the needle cover reaches its retracted position. Hence, when the user presses the needle cover against the skin, the needle cover is pushed into the drive unit housing and when the needle cover reaches its retracted position, the trigger button is unlocked and lifts out of the drive unit housing to its extended position and can be pressed by the user.

Preferably, the drive unit further comprises a trigger button spring to bias the trigger button towards its extended position. Hence, the trigger spring automatically moves the trigger button to its extended position, once the trigger button is released by the needle cover reaching its proximal retracted position.

Preferably, the drive unit further comprises a trigger arm operationally coupled to the needle cover, wherein the trigger arm comprises a recess and the trigger button comprises a protrusion. When the needle cover is in its retracted position, the protrusion is received in the recess, locking the trigger button in its retracted position. Hence, the trigger button is fixed inside the drive unit housing until the user removes the drug delivery device from the injection site. This prevents a partial release of the set dose in case the user accidentally stops pressing the trigger button down during the injection. It is also more comfortable for the user not having to press down the trigger button during the entire injection.

Preferably, the drive unit further comprises a lockout arm movable between a radial inner position and a radial outer position. In the inner position the needle cover is movable in the proximal direction and in the outer position the lockout arm prevents the needle cover from moving in the proximal direction. The plunger rod comprises a projection on its proximal end that moves the lockout arm from the inner position to the outer position when the plunger rod reaches its distal position. This provides an end of dose stop so that the needle cover cannot be pushed into the drive unit housing to start an injection, when an insufficient amount of medicament for the set dose is remaining in the reservoir.

The invention also relates to a drug delivery device comprising an inventive drive unit and a reservoir releasably attachable to the drive unit. Preferably, the drive unit housing, the plunger rod, the drive nut, the stepped drum, the cap, the priming rod, the priming gear, the rack, the helical gear, the check tab, the trigger button, the needle cover, the priming sleeve, the loading sleeve, the plunger rod shuttle and/or the plunger rod reset sleeve are injection-molded thermoplastic plastic parts. The injection-molded parts allow for a cost-effective production of the drug delivery device parts in particular in case of a high-volume production.

### BRIEF DESCRIPTION OF THE DRAWINGS

The subject matter of the invention will be explained in more detail in the following text with reference to preferred example embodiments which are illustrated in the attached drawings, in which depict:
- Fig. 1a - d: a first example embodiment of an inventive drive unit of a drug delivery device in different dose settings;
- Fig. 2: a perspective view of a middle part of the drive unit of fig. 1a - d;
- Fig. 3: a cross-sectional view of the drive unit of fig. 1a to 2 along line III - III in fig. 2;
- Fig. 4: the drive unit of fig. 1a to 3 during an injection;
- Fig. 5: a perspective view of the plunger rod, the drive nut and the stepped drum of the drive unit of fig. 1a to 4;
- Fig. 6: the drive unit of fig. 1a to 5 after the injection;
- Fig. 7: another perspective view of the drive unit of fig. 1a to 6;
- Fig. 8: a cross-sectional view of the plunger rod, the drive nut and the stepped drum of the drive unit of fig. 1a to 7 at the end of an injection;
- Fig. 9: a second example embodiment of an inventive drive unit of a drug delivery device in an initial, unloaded state;
- Fig. 10: the drive unit of fig. 9 while the mechanical drive is loaded;
- Fig. 11: the drive unit of fig. 9 and 10 with the mechanical drive fully loaded;
- Fig. 12: the drive unit of fig. 9 to 11 after an injection;
- Fig. 13: a schematic representation of the needle cover movement of the drive unit of fig. 9 to 12 during loading of the mechanical drive and during the injection;
- Fig. 14: a perspective view of a distal part of the drive unit of fig. 9 to 13 with the plunger rod reset sleeve in the second position;
- Fig. 15: a perspective view of a distal part of the drive unit of fig. 9 to 14 with the plunger rod reset sleeve in the first position;
- Fig. 16a - c: the drive unit of fig. 9 to 15 in different dose settings;
- Fig. 17: a third example embodiment of an inventive drive unit of a drug delivery device in an initial position;
- Fig. 18: the drive unit of fig. 17 with the trigger button in a retracted position;
- Fig. 19: the drive unit of fig. 17 and 18 with the trigger button in the extended position;
- Fig. 20: the drive unit of fig. 17 to 19 with the trigger button pressed to start the injection;
- Fig. 21: the drive unit of fig. 17 to 20 after the injection; and
- Fig. 22: a perspective view of the plunger rod and the stop element of the drive unit of fig. 17 to 21 in different dose settings.

The reference symbols used in the drawings, and their primary meanings, are listed in summary form in the list of designations. In principle, identical parts are provided with the same reference symbols in the figures.

### DETAILED DESCRIPTION OF THE PREFFERRED EMBODIMENTS

In the present description the term "distal" refers to the side where the injection needle is located and the term "proximal" refers to the opposite side or rear end of the device.

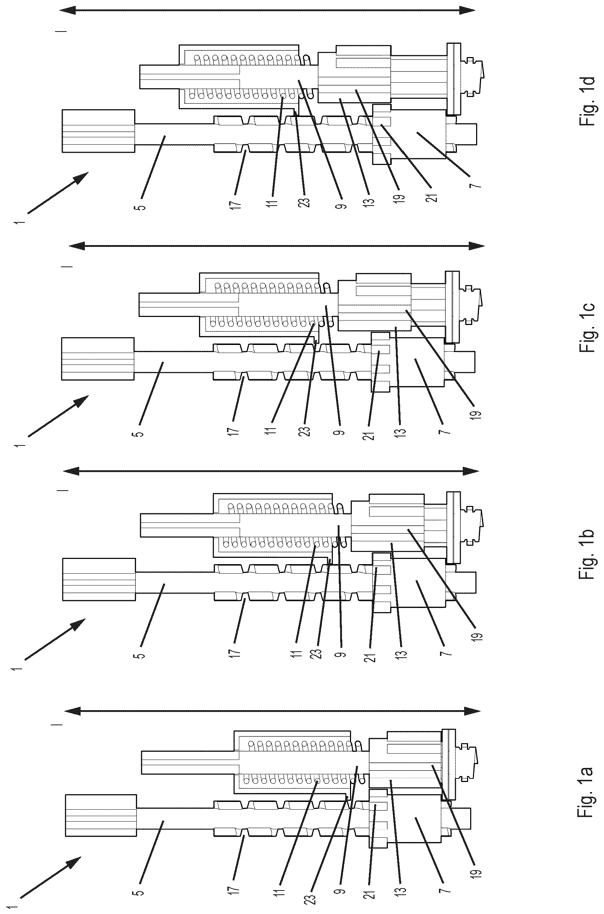

Fig. 1a to 8 show a first example embodiment of a drive unit 1 of a drug delivery device of the present disclosure. The drive unit 1 is releasably attachable to a reservoir (not shown in the figures) to provide a complete drug delivery device. The drive unit 1 comprises a drive unit housing (not shown in fig. 1a to 8) defining a longitudinal direction l.

Inside the drive unit housing, the drive unit 1 comprises a plunger rod 5 that is movable in a dispensing direction to expel medicament from the reservoir. In this embodiment, the dispensing direction is the longitudinal direction l, specifically the distal direction. The plunger rod 5 cooperates with a drive nut 7 that is arranged near the distal end of the plunger rod 5. The drive unit 1 further comprises a priming rod 9 arranged parallel to the plunger rod 5. The priming rod 9 is connected to one end of a mechanical drive 11, so that the mechanical drive 11 is loaded, when the priming rod 9 rotates. In this embodiment, the mechanical drive 11 is a torsion spring. The other end of the mechanical drive 11 is connected to a stepped drum 13 that cooperates with the drive nut 7. During an injection, the loaded mechanical drive 11 rotates the stepped drum 13 that rotates the drive nut 7. Therefore, the stepped drum 13 comprises a plurality of teeth 19 and the drive nut 7 also comprises a plurality of teeth 21. The drive nut 7 further comprises an inner thread 15 (not shown in fig. 1) and the plunger rod 5 comprises an outer thread 17, so that the rotation of the drive nut 7 is translated or transferred into an axial movement of the plunger rod 5 in the dispensing direction to dispense the drug. After the injection, the mechanical drive 11 can be reloaded by rotating the priming rod 9.

Fig. 1a - d show the drive unit 1 in different doses settings. The dose can be set by a dose setting mechanism that comprises a trigger element and a dose setting element. The dose setting element sets the dose and the trigger element serves to load the mechanical drive upon a loading stroke of the trigger element. In this embodiment, the dose setting element is the stepped drum 13 and the trigger element is a cap of the drive unit 1 (not shown in fig. 1a - d). To set different doses, the stepped drum 13 is movable relative to the drive nut 7 in the longitudinal direction l.

In fig. 1a, the stepped drum 13 is in its most distal position and in fig. 1d the stepped drum 13 is in its most proximal position. To set different doses with the longitudinal movement of the stepped drum 13, the teeth 19 of the stepped drum 13 have different length in longitudinal direction l. In fig. 1a - d, on the left side, the stepped drum 13 comprises two teeth that extend along the entire length of the stepped drum 13 and two teeth on the left side that extend along ¾ of the length of the stepped drum 13. On the back side of the stepped drum 13, not visible in fig. 1a to 1d, the stepped drum 13 further has two teeth extending along half of the length of the stepped drum 13 and two teeth extending along ¼ of the length of the stepped drum 13. When the stepped drum 13 is in its most distal position shown in fig. 1a, only the two longest teeth engage with the teeth 21 of the drive nut 7, so that for a full rotation of the stepped drum 13, the drive nut only makes ¼ of a full rotation. This means, that the plunger rod 5 also only moves ¼ of the full dispensing stroke. In fig. 1b, the stepped drum 13 is moved in the distal direction so that two more teeth 19 of the stepped drum 13 engage with the teeth 21 of the drive nut 7 and the drive nut 7 fulfills half a rotation for a full rotation of the stepped drum 13. In fig. 1c, the stepped drum 13 is moved further in the distal direction so that again two more teeth 19 of the stepped drum 13 engage with the teeth 21 of the drive nut 7 and finally in fig. 1d, with the stepped drum 13 in its most proximal position, all teeth 19 of the stepped drum 13 engage with the drive nut 7 so that the drive nut 7 makes a full rotation when the stepped drum 13 makes a full rotation. The set dose can for example be 0.25 ml in fig. 1a, 0.5 ml in fig. 1b, 0.75 ml in fig. 1c and 1 ml in fig, 1d. In fig. 1, the stepped drum 13 is part of a subassembly that is moved in its entirety relative to the rest of the drive unit 1 to set different doses. The subassembly further comprises the mechanical drive 11 and a check tab 23, that will be explained in detail below.

With the dose setting mechanism shown in fig. 1a - d, it is possible to dispense different doses of the medicament while the loading stroke of the trigger element remains the same. In this embodiment, the loading of the mechanical drive 11 also remains the same in that the mechanical drive 11 is fully loaded with each loading stroke of the trigger element and fully unloaded after the end of each injection.

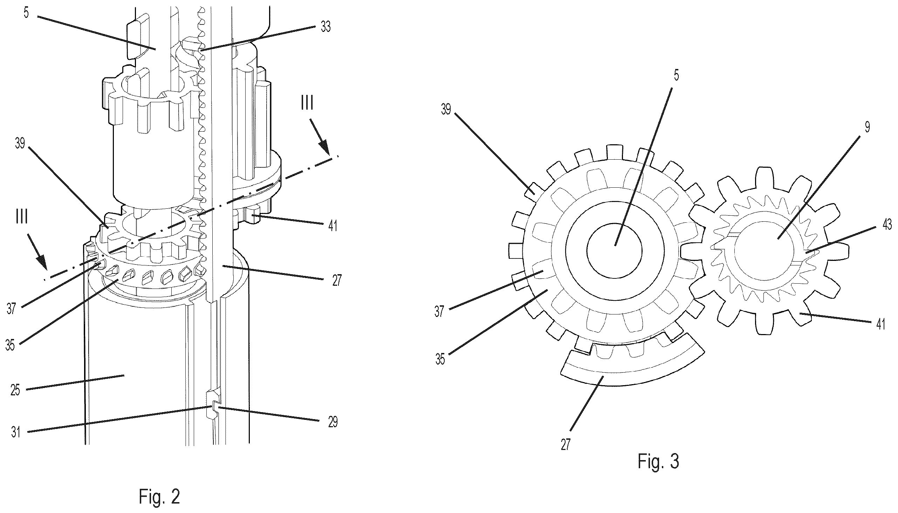

Fig. 2 and 3 show how the mechanical drive 11 can be loaded, in that Fig. 2 is a perspective view of a middle part of the drive unit 1 of fig. 1a - d and Fig. 3 is a cross-sectional view of the drive unit 1 along line III - III in fig. 2.

As mentioned before, the mechanical drive 11 can be loaded by a loading stroke of the trigger element. In this embodiment the trigger element is the cap 25 and the loading stroke is a distal movement of cap 25 to remove the cap 25 of the drive unit 1 before the injection. Cap 25 is connected to a rack 27 by a cap clip 29 and a respective recess 31 in the rack 27. The rack 27 comprises teeth 33 that cooperate with a helical gear 35 arranged on the plunger rod 5 distal of the drive nut 7. The helical gear 35 comprises helical teeth 37 to translate or transfer the axial movement of the rack 27 into a rotation of the helical gear 35. For better force transmission, multiple helical teeth 37 engage with the teeth 33 of the rack 27 at the same time. The helical gear 35 further comprises regular teeth 39 that cooperate with a priming gear 41 that is connected to the priming rod 9 with a ratchet feature 43. The ratchet feature 43 is adapted to translate the rotation of the priming gear 41 to a rotation of the priming rod 9, when the priming gear 41 rotates in a first direction and to let the priming gear 41 rotate independently from the priming rod 9, when the priming gear 41 rotates in the opposite direction. The first rotation direction correlates to a distal movement of the cap 25, so that the priming rod 9 rotates when the helical gear 35 rotates due to the removal of the cap 25 which loads the mechanical drive 11. In Fig.3, the helical gear 35 rotates anticlockwise and the priming gear 41 and the priming rod 9 rotate clockwise, as the ratchet feature 43 is engaged for this rotation direction of the helical gear 35.

As explained before, in this embodiment, for each injection the mechanical drive 11 is fully loaded. When the mechanical drive 11 is fully loaded, the priming rod 9 and the stepped drum 13 are latched to keep the mechanical drive 11 in the loaded state. After the cap 25 has completed the loading stroke and the mechanical drive 11 is fully loaded, the cap clip 29 radially deforms outwards so that the clip 29 disengages from the rack 27 allowing removal of the cap 25 from drive unit 1.

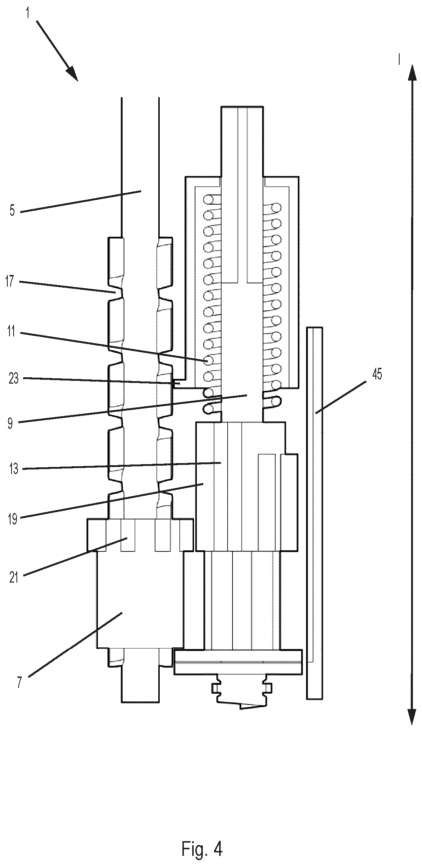

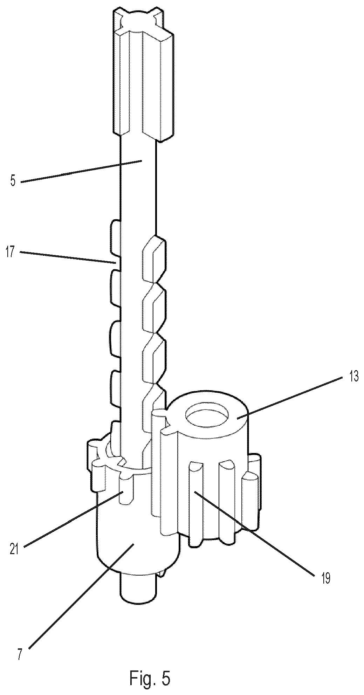

Fig. 4 and 5 illustrate the injection triggering and the drug delivery process of the drive unit 1. After the mechanical drive 11 is fully loaded, the user can press a trigger button (not shown in fig. 4 and 5) to start the injection. The trigger button comprises or is connected to a trigger button arm 45 illustrated in fig. 4 that latches the stepped drum 13 until the trigger button is pressed. When the user presses the trigger button, arm 45 moves in the distal direction and releases the stepped drum 13 so that the stepped drum 13 is free to rotate under the bias of the loaded mechanical drive 11. The mechanical drive 11 drives the stepped drum 13 for a full rotation, with other words a 360° rotation. Depending on the dose setting, a different number of teeth 19 of the stepped drum 13 engage with the teeth 21 of the drive nut 7, resulting in a partial rotation of the drive nut 7. The rotation of the drive nut 7 is translated or transferred into a distal movement of the plunger rod 5 through the threaded connection between the drive nut 7 and the plunger rod 5 to dispense the medicament. Fig. 5 shows the stepped drum 13 in a perspective view in which six teeth 19 of the stepped drum 13 with three different axial length can be seen.

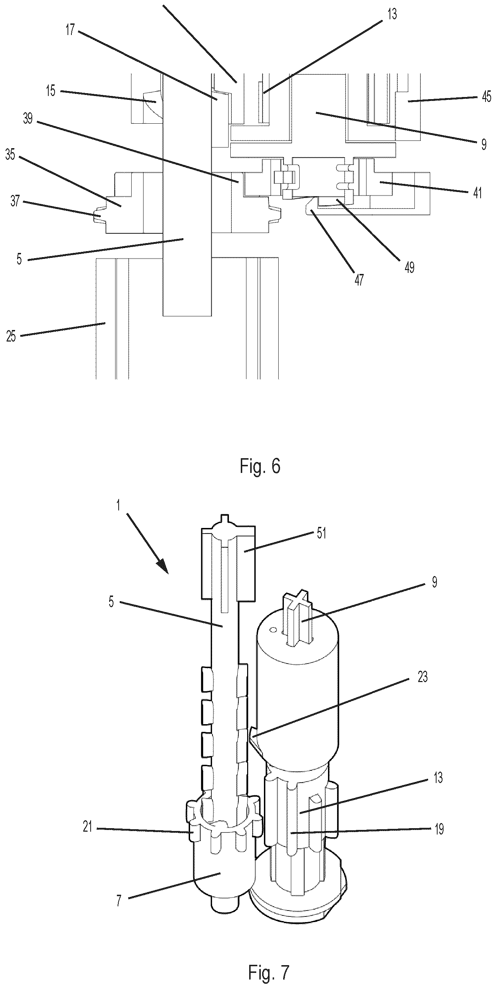

Fig. 6 is a cross-sectional view of the distal part of the drive unit 1 that illustrates how cap 25 can be reattached after an injection and how drive unit 1 can be reset. After delivering a dose, the user pushes the cap 25 back onto drive unit 1 which pushes the rack 27 (not shown in fig. 6) in the proximal direction back to its starting position. Doing so drives the helical gear 35 and the priming gear 41 in reverse. Due to the ratchet feature 43, the priming rod 9 is not rotated so that the user can put the cap 25 back on without loading the mechanical drive 11 and hence without the need to exert the force to load the mechanical drive 11. Fig. 6 further shows a preload ratchet 47 at the distal end of the priming rod 9 that runs up a ramp feature 49 on the distal end surface of the priming rod 9, when the cap 25 is removed. In the event of the cap 25 being only partially removed and then reattached to drive unit 1, the preload ratchet 47 allows the priming rod 9 to return to its rest position, preventing partial loading of the mechanical drive 11. In the embodiment of fig. 6, the preload ratchet 47 is a separate part but in other embodiments the preload ratchet 47 could be integrated into the drive unit housing.

Fig. 7 illustrates how drive unit 1 prevents the user from setting a dose if there is insufficient medicament remaining in the reservoir. If there is sufficient dose to deliver the current dose setting, the check tab 23 is uninhibited and the priming rod 9 can be rotated to load the mechanical drive 11. This situation is shown in fig. 7. However, if there is an insufficient dose remaining, the plunger rod 5 is in a more distal position causing the check tab 23 to abut a protrusion 51 on the proximal end of the plunger rod 5, so that the check tab 23 and the priming rod 9 connected to the check tab 23 cannot be rotated. Then the user cannot fully remove the cap 25 unless the dose is reduced to a valid amount.

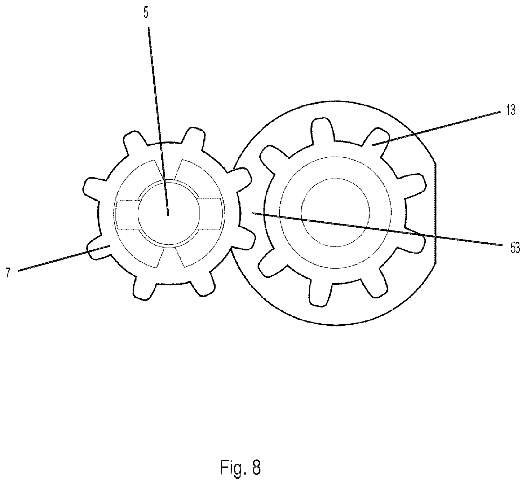

Fig. 8 illustrates how the drive unit 1 can be reset after the reservoir is empty. In this case the user first removes the empty reservoir and then pushes the plunger rod 5 back to its proximal position. The plunger rod 5 can for example be pushed back when the user inserts a new reservoir. The axial movement of the plunger rod 5 rotates the drive nut 7 in the opposite direction than during the injection. To prevent a loading of the mechanical drive 11 through a rotation of the stepped drum 13 that would require the user to exert the force necessary to load the mechanical drive 11 while pushing back the plunger rod 5, the stepped drum 13 comprises a gap 53 between two teeth 19, in this case between the longest teeth and the shortest teeth of the stepped drum 13. When the injection is finished and the stepped drum 13 has made a full rotation, the gap 53 faces the drive nut 7 so that the drive nut 7 is free to rotate without rotating the stepped drum 13.

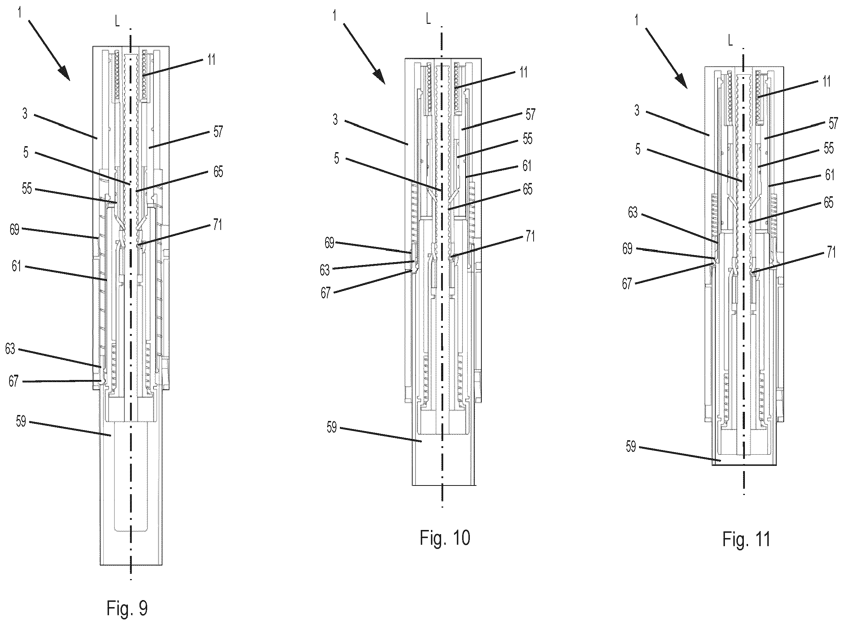

Fig. 9 to 16c show a second example embodiment of a drive unit 1 of a drug delivery device of the present disclosure that is releasably attachable to a reservoir.

Drive unit 1 comprises a drive unit housing 3 defining a longitudinal direction respectively a longitudinal axis L. Inside the drive unit housing 3, the drive unit 1 comprises a plunger rod 5 that is movable in a dispensing direction to expel the medicament from the reservoir. In this embodiment, the dispensing direction is along the longitudinal axis L, specifically in the distal direction. The plunger rod 5 is pushed in the dispensing direction by a plunger rod shuttle 55 that cooperates with a loading sleeve 57 that is rotated by a loaded mechanical drive 11. In this embodiment, the mechanical drive 11 is a torsion spring. To set a dose, the drive unit 1 further comprises a dose setting mechanism with a dose setting element and a trigger element. In this embodiment, the trigger element is a needle cover 59 that is movable between an extended position in which it covers the needle and a retracted position in which the needle is revealed to perform an injection. When the needle cover 59 moves from the extended position to the retracted position it pushes a priming sleeve 61 in the proximal direction. The proximal movement of the priming sleeve 61 causes a rotation of the loading sleeve 57 through a helical track between the priming sleeve 61 and the loading sleeve 57. The detailed mechanism to load the mechanical drive 11 and to dispense a dose will be explained in detail below.

Fig. 9 shows the drive unit 1 in an initial state with the needle cover 59 in its extended distal position. Fig. 10 shows the drive unit 1 with the needle cover 59 moved proximally from the extended position but the needle cover 59 has not reached the retracted position. The proximal movement of the needle cover 59 is caused partly by attaching the needle to the reservoir and partly by pressing the needle cover 59 against the skin at a desired injection site, which will be explained in detail later. When the needle cover 59 moves proximally, it also pushes the priming sleeve 61 and a dose release collar 63 in the proximal direction. The proximal movement of the priming sleeve 61 rotates the loading sleeve 57 that is connected to one end of the mechanical drive **11** and therefore loads the mechanical drive 11. The rotation of the loading sleeve 57 also moves the plunger rod shuttle 55 in the proximal direction. The plunger rod shuttle 55 thereby slides over ramped projections 65 on the plunger rod 5 so that the plunger rod 5 is not moved in the proximal direction. Fig. 11 shows the drive unit 1 with the needle cover 59 in the retracted position. In this position, the mechanical drive 11 is fully loaded and arms 67 of the dose release collar 63 are no longer reinforced by the wall of the drive unit housing 3 so that they deflect outwards into recesses 69 in the drive unit housing 3. Arms 67 thereby release the priming sleeve 61, allowing the mechanical drive 11 to rotate the loading sleeve 57. This instantaneous release of the mechanical drive 11 makes a click sound that indicates the start of the injection.

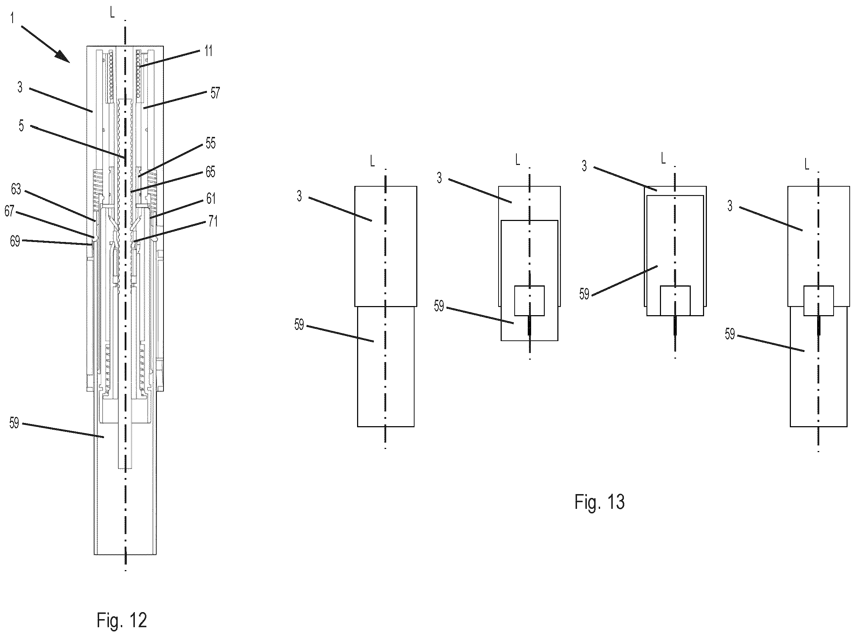

Fig. 12 shows the drive unit 1 during an injection. During the injection, the plunger rod shuttle 55 is driven in the dispensing direction by the rotation of the loading sleeve 57 caused by the loaded mechanical drive 11. Thereby, the plunger rod shuffle 55 pushes the plunger rod 5 in the dispensing direction due to flat proximal surfaces of the ramped protrusions 65 of the plunger rod 5 to dispense the medicament from the reservoir. The drive unit 1 further comprises click arms 71 that produce continuous clicking while the plunger rod protrusions 65 pass the click arms 71. At the end of the dose delivery, the loading sleeve 57 reaches a rotational end stop and ceases motion. The user can then remove the needle cover 59 from the skin so that the needle cover 59 moves back to its extended position. This allows the dose release collar 63 to also move back in the distal direction, whereby arms 67 are deflected back inwards and abut the wall of the drive unit housing 3 again.

Fig. 13 summarizes the movement of the needle cover 59 from the initial extended position to an intermediate position by attaching the needle to the reservoir, to the retracted position by pushing the needle cover 59 against the skin at a desired injection site and back to the initial extended position, when the user removes the drug delivery device from the skin after the injection. In this embodiment, the needle cover 59 has dual use as trigger element of the dose setting mechanism and to trigger the injection. The movement of the needle cover can be guided by a pin and track mechanism (not shown in fig. 13) established between the drive unit housing and the needle cover respectively the dose release collar.

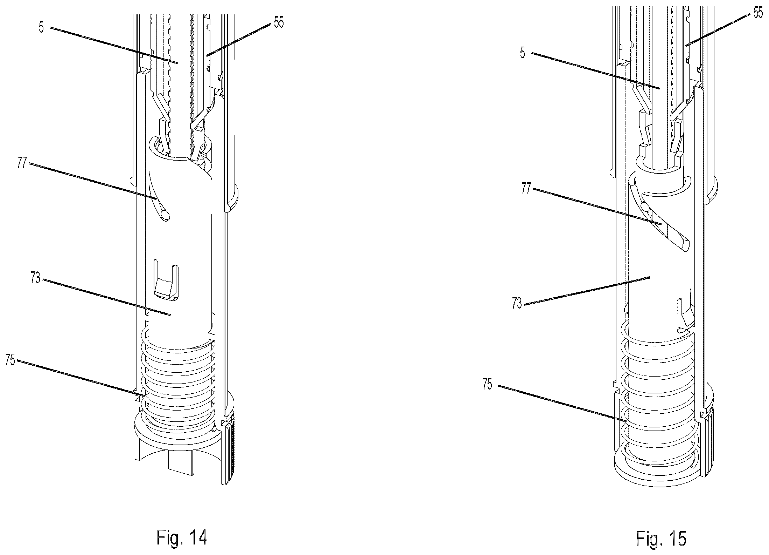

Fig. 14 and 15 illustrate how the plunger rod 5 can be reset after the reservoir is empty. Fig. 14 shows the drive unit 1 when a reservoir is attached to the drive unit 1. The reservoir pushes a plunger rod reset sleeve 73 in the proximal direction, which keeps the plunger rod 5 in engagement with the plunger shuttle 55 so that the plunger rod shuttle 55 can advance the plunger rod 5 to dispense the medicament. When the reservoir is removed from the drive unit 1, the plunger rod reset sleeve 73 moves in the distal direction under the bias of a reset spring 75 as shown in fig. 15. The plunger rod reset sleeve 73 comprises a helical track 77 that rotates the plunger rod 5 when the plunger rod reset sleeve 73 moves in the distal direction. Due to this the plunger rod 5 no longer engages the plunger rod shuttle 55, so that the plunger rod 5 can be freely pushed back into the drive unit housing 3 in the proximal direction.

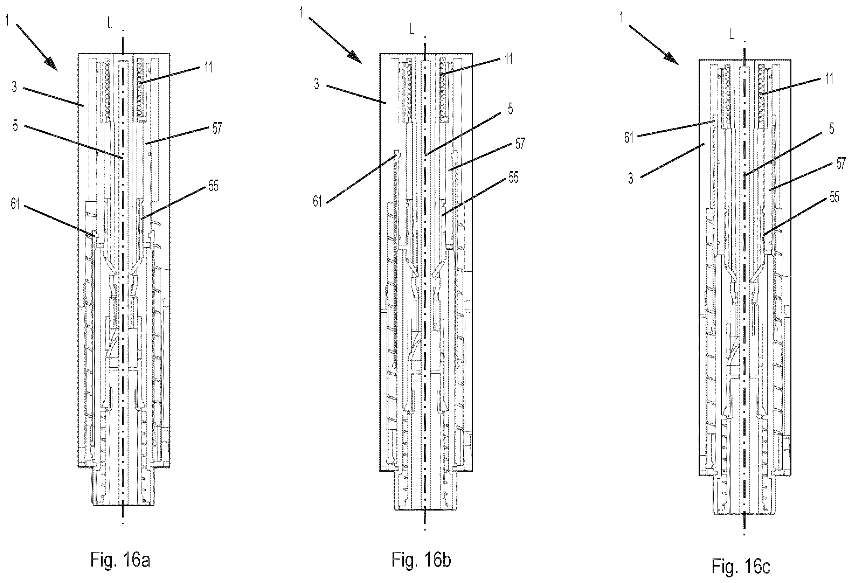

Fig. 16a - c show how different doses can be set by moving the priming sleeve 61 in the longitudinal direction respectively along the longitudinal axis L. In fig. 16a, the priming sleeve 61 is in its most distal position, in fig. 16b in an intermediate position and in fig. 16c in its most proximal position. The position in fig. 16a can correspond to a dose of 1 ml, the position in fig. 16b to a dose of 0.5 ml and the position in fig. 16c to a dose of 0.25 ml. The movement of the priming sleeve 61 changes the distance between a distal end of the priming sleeve 61 and a proximal end of the needle cover 59 (not shown in fig. 16a - c) in its initial extended position. Hence, during the loading stroke of the needle cover 59, the needle cover 59 moves over an altering distance before reaching the distal end of the priming sleeve 61 and subsequently pushing the priming sleeve 61 in the proximal direction to load the mechanical drive 11. This means that with the same loading stroke of the needle cover 59, the mechanical drive 11 is loaded to a different amount depending on the position of the priming sleeve 61 relative to the needle cover 59. The priming sleeve 61 is therefore the dose setting element in this embodiment. During the injection, the mechanical drive 11 is then fully unloaded.

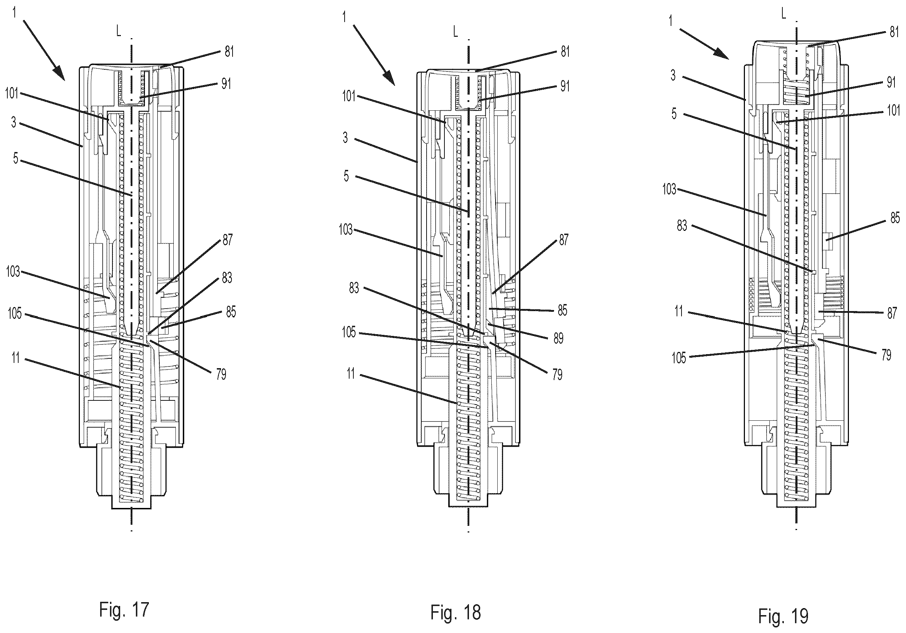

Fig. 17 to 22 show a third example embodiment of a drive unit 1 of a drug delivery device of the present disclosure that is releasably attachable to a reservoir.

Fig. 17 shows the drive unit 1 in a pre-delivery state in which the mechanical drive 11 is loaded but the plunger rod 5 is prevented from moving in the distal direction by a stop element 79 abutting an end stop 83 of the plunger rod 5. Hence, the loaded mechanical drive 11 cannot push the plunger rod 5 in the distal direction. In this position, the trigger button 81 is held in its retracted position inside the drive unit housing 3 so that the user cannot press the trigger button 81 to start the injection. Fig. 18 shows the drive unit 1 during unlocking of the trigger button 81. To unlock the trigger button 81, the user presses the drug delivery device against the skin so that the needle cover (not shown in fig. 18) is moved to its proximal retracted position. The needle cover thereby raises a needle cover shuttle 85 that also moves in the proximal direction. The needle cover shuttle 85 pushes a trigger arm 87 radially outwards. For this purpose, the needle cover shuttle 85 comprises a wedge 89. In Fig. 19, the drive unit 1 is fully unlocked. In this position, the trigger arm 87 no longer engages the stop element 79. This allows the trigger button 81 to move into its extended position under the bias of a trigger button spring 91. In this extended position, the user can press the trigger button 81 to start the injection.

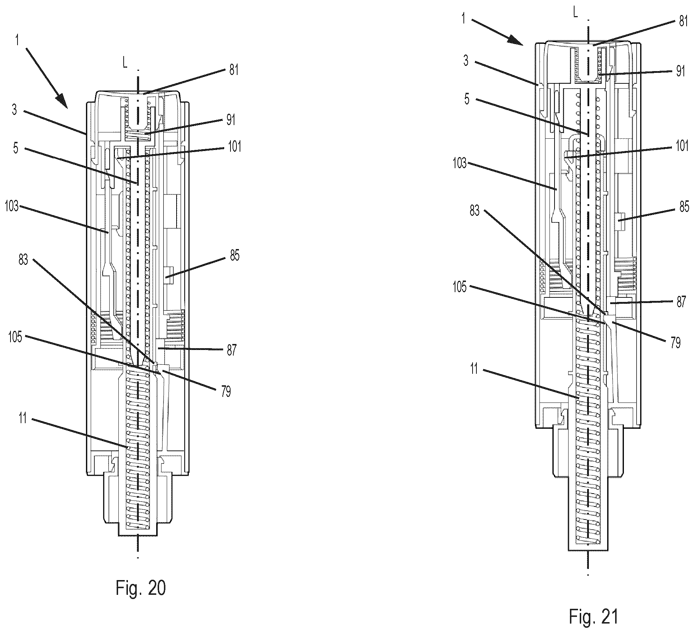

Fig. 20 shows the drive unit 1 after a dose is initiated by pressing the trigger button 81. When the trigger button 81 is depressed, the trigger arm 87 moves in the distal direction and pushes the stop element 79 radially outwards. This releases the plunger rod 5, because the stop element 79 no longer abuts the end stop 83. The plunger rod 5 can hence move in the distal direction to dispense the set dose under the bias of the loaded mechanical drive 11. After a distal end of the trigger arm 87 has passed the stop element 79, the stop element 79 returns back to its radial inner position, as shown in fig. 21. The stop element 79 then fixes the trigger arm 87 and thereby keeps the trigger button 81 in its retracted position inside the drive unit housing 3. The doses delivery ceases, when the next end stop 83 of the plunger rod 5 abuts the stop element 79, so that the plunger rod 5 cannot move further in the distal direction. The user then removes the drug delivery device from the injection site which allows the needle cover and the needle cover shuttle 85 to move in the proximal direction to their initial positions shown in fig. 17.

The plunger rod 5 further comprises a lockout projection 101 on its proximal end. When the plunger rod 5 reaches its most distal position, the lockout projection 101 pushes a lockout arm 103 radially outwards. The lockout arm 103 then prevents a proximal movement of the needle cover so that it is not possible to perform another injection. The user then must replace the reservoir and push the plunger rod 5 back in the proximal direction to reload the mechanical drive. To enable the proximal movement of the plunger rod 5, the stop element 79 comprises a ramped distal surface 105 that slides over the end stops 83 of the plunger rod 5, when the end stops 83 pass the stop element 79 and thereby deflect the stop element 79 outwards.

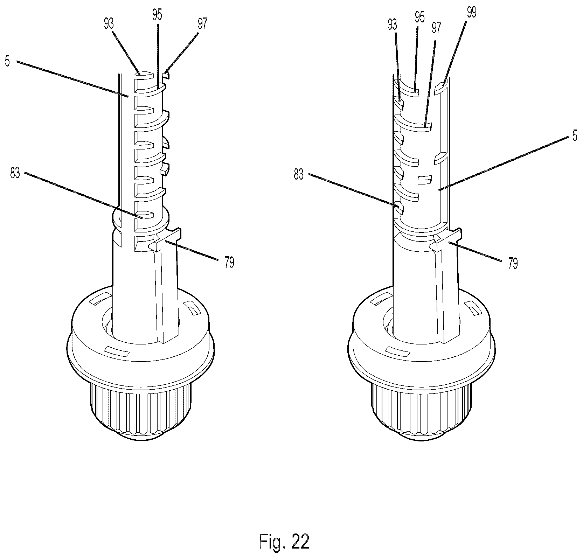

Fig. 22 shows the dose setting mechanism of the drive unit 1 of fig. 17 to 22. The dose setting mechanism comprises a trigger element and a dose setting element. In this embodiment, the trigger element is the plunger rod 5 that is pushed in the proximal direction by the user to load the mechanical drive 11, when the user attaches a new reservoir to the drive unit 1. The dose setting element is formed by tracks 93, 95, 97, 99 on the plunger rod 5. Hence, in this embodiment, the plunger rod 5 provides the entire dose setting mechanism. Each track 93, 95, 97, 99 comprises a number of end stops 83 with a specific distance in the longitudinal direction between them. The distance between two end stops 83 is different for each track 93, 95, 97, 99. Hence, the distance the plunger rod 5 can travel until the next end stop 83 abuts the stop element 79 is different for each track 93, 95, 97, 99 so that each track 93, 95, 97, 99 corresponds to a different set dose. By rotating the plunger rod 5 and the stop element 79 relative to each other, the user can change the track 93, 95, 97, 99 engaging with the stop element 79 and thereby set a desired dose. On the left side of fig. 22, track 95 is aligned with stop element 79. Track 95 can correspond to a set dose of 0.5 ml and the distance between two end stops 83 of track 95 can be 6.766 mm for a 3 ml cartridge with an inner diameter of 9.7 mm. On the right side of fig. 22, track 99 is aligned with the stop element 79. Track 99 can correspond to a set dose of 1 ml and the distance between two end stops 83 of track 99 can be 13.532 mm for a 3 ml cartridge with an inner diameter of 9.7 mm.

The previous description of the disclosed embodiments is provided to enable a person skilled in the art to make or use the disclosed embodiments. Various modifications to these embodiments will be readily apparent to those skilled in the art, and the principles defined herein may be applied to other embodiments without departing from the scope of the disclosure. Thus, the present disclosure is not intended to be limited to the embodiments shown herein but is to be accorded the widest scope possible consistent with the principles and novel features as previously shown and described.

### LIST OF DESIGNATIONS

- 1: drive unit
- 3: drive unit housing
- 5: plunger rod
- 7: drive nut
- 9: priming rod
- 11: mechanical drive
- 13: stepped drum
- 15: inner thread drive nut
- 17: outer thread plunger rod
- 19: teeth stepped drum
- 21: teeth drive nut
- 23: check tab
- 25: cap
- 27: rack
- 29: cap clip
- 31: rack recess
- 33: teeth rack
- 35: helical gear
- 37: helical teeth
- 39: regular teeth helical gear
- 41: priming gear
- 43: ratchet feature
- 45: trigger button arm
- 47: preload ratchet
- 49: ramp feature
- 51: plunger rod protrusion
- 53: gap
- 55: plunger rod shuttle
- 57: loading sleeve
- 59: needle cover
- 61: priming sleeve
- 63: dose release collar
- 65: ramped projection
- 67: arms
- 69: recess
- 71: click arms
- 73: plunger rod reset sleeve
- 75: reset spring
- 77: helical track
- 79: stop element
- 81: trigger button
- 83: end stop
- 85: needle cover shuttle
- 87: trigger arm
- 89: wedge
- 91: trigger button spring
- 93 - 99: tracks
- 101: lockout projection
- 103: lockout arm
- 105: ramped distal surface

- l: longitudinal direction
- L: longitudinal axis

## Claims

1. Drive unit (1) of a drug delivery device for dispensing predefined doses of a liquid drug, releasably attachable to a reservoir and comprising,
- a drive unit housing (3) defining a longitudinal direction (l), a plunger rod (5) movable inside the drive unit housing (3) in a dispensing direction to dispense the liquid drug and a mechanical drive (11), preferably a torsion spring, arranged inside the drive unit housing (3) for moving, when loaded, the plunger rod (5) in the dispensing direction; and
- a dose setting mechanism comprising a dose setting element (13; 61) and a trigger element (25; 59), wherein a loading stroke of the trigger element (25; 59) causes the dose setting mechanism to load the mechanical drive (11);
wherein the dose setting element (13; 61) is movable to change a stroke distance of the plunger rod (5) such that the predefined dose is changeable while the loading stroke of the trigger element (25; 59) remains the same.

2. Drive unit (1) according to claim 1, wherein the mechanical drive (11) is fully loaded with each loading stroke of the trigger element (25) and the dose setting element (13) changes the stroke distance of the plunger rod (5) by altering the transmission between the mechanical drive (11) and the plunger rod (5).

3. Drive unit (1) according to claim 1 or 2, further comprising a drive nut (7) and a stepped drum (13) for transferring force from the mechanical drive (11) to a movement of the plunger rod (5) in the dispensing direction, wherein the stepped drum (13) is the dose setting element and comprises teeth (19) of different longitudinal length, wherein the stepped drum (13) is movable relative to the drive nut (7) in the longitudinal direction (l) in such a way that the number of teeth (19) of the stepped drum (13) engaging with teeth (21) of the drive nut (7) is altered.

4. Drive unit (1) according to claim 3, wherein the stepped drum (13) comprises a gap (53) between at least two adjacent teeth (19) such that the drive nut (7) can freely rotate during reset of the drug delivery device.

5. Drive unit (1) according to one of the preceding claims, wherein the trigger element is a cap (25) and the dose setting mechanism is adapted to load the mechanical drive (11) upon a distal movement of the cap (25).

6. Drive unit (1) according to claim 5, wherein the mechanical drive (11) is arranged on a priming rod (9) coupled to a priming gear (41), the priming rod (9) being rotatable to load the mechanical drive (11), and wherein the cap (25) is coupled to a rack (27) cooperating with a helical gear (35), wherein the helical gear (35) cooperates with the priming gear (41).

7. Drive unit (1) according to claim 6, wherein the priming gear (41) and/or the priming rod (9) has a ratchet feature (43) so that the priming gear (41) and the priming rod (9) rotate together when the priming gear (41) is rotated in a first direction and the priming gear (41) rotates without rotating the priming rod (9) when the priming gear (41) rotates in an opposite direction and/or wherein the priming rod (9) is rotationally coupled to a check tab (23) movable together with the stepped drum (13) and the plunger rod (5) comprises a protrusion (51) on its proximal end that is adapted to collide with the check tab (23) when a dose remaining in the reservoir is insufficient for the set dose.

8. Drive unit (1) according to one of the preceding claims, further comprising a trigger button to start the injection, preferably on a proximal end of the drive unit (1), wherein preferably the trigger button comprises a ramped proximal surface and/or the trigger button comprises a rotational end stop abutting the stepped drum (13) after a full rotation of the stepped drum (13).

9. Drive unit (1) according to claim 8, wherein the priming rod (9) and/or the check tab (23) comprises a protrusion that limits the trigger button from being pressed before the mechanical drive (11) is loaded and/or a protrusion that locks the trigger button in a pressed-in position during the injection.

10. Drive unit (1) according to claim 1, wherein the mechanical drive (11) is partly or fully loaded with each loading stroke of the trigger element dependent on the position of the dose setting element.

11. Drive unit (1) according to claim 1 or 10, wherein the trigger element is a needle cover (59) movable between an extended position in which it covers the needle and a retracted position in which it reveals the needle, wherein the dose setting mechanism is adapted to load the mechanical drive (11) by a proximal movement of the needle cover (59) from the extended position to the retracted position.

12. Drive unit (1) according to claim 11, wherein the needle cover (59) is movable from the extended position to an intermediate position in which it still covers the needle by attaching the needle and from the intermediate position to the retracted position by pressing the needle cover (59) against the skin.

13. Drive unit (1) according to one of claims 1, 10 or 11, wherein the dose setting element is a priming sleeve (61) movable together with the needle cover (59) in the proximal direction, wherein the priming sleeve (61) comprises a thread for translating its proximal movement to a rotation of a loading sleeve (57) that loads the mechanical drive (11), wherein the priming sleeve (61) is movable relative to the needle cover (59) in the longitudinal direction (l) in such a way that a distance between a proximal end of the needle cover (59) in the extended position and the priming sleeve (61) is altered.

14. Drive unit (1) according to claim 13, further comprising a plunger rod shuttle (55) comprising a thread to cooperate with the loading sleeve (57) and engaging with the plunger rod (5) to translate rotation of the loading sleeve (57) to a movement of the plunger rod (5) in the dispensing direction.

15. Drive unit (1) according to claim 14, further comprising a plunger rod reset sleeve (73) movable between a first extended position and second retracted position by inserting the reservoir, wherein in the first position, the plunger rod (5) is separated from the plunger shuttle (55) and in the second position, the plunger rod (5) is coupled to the plunger shuttle (55), wherein the plunger rod reset sleeve (73) comprises a helical ramp (77) to rotate the plunger rod (5) while moving between the first position and the second position.
